# FASCICULE DE BREVET EUROPEEN

(11) **EP 0 882 979 B1**
(45) Date de publication et mention de la délivrance du brevet: **25.08.2004**
(21) Numéro de dépôt: 98401344.1
(22) Date de dépôt: 04.06.1998
(51) Int. Cl.: G01N 27/327, G01N 33/543

(54) **Support d'électrodes comportant au moins une électrode recouverte par un dépot et système de lecture de ce support**
Elektrodentragvorrichtung bestehend aus mindestens einer Elektrode mit Überzug und System zum lesen
Electrode holder comprising at least one covered electrode and reading-out system

(30) Priorité: 06.06.1997 FR 9707048
(43) Date de publication de la demande: 09.12.1998
(73) Titulaire: COMMISSARIAT A L'ENERGIE ATOMIQUE, 75015 Paris (FR)
(72) Inventeur: Clerc, Jean-Frédéric, 38120 Le Fontanil (FR)
(74) Mandataire: Poulin, Gérard

(56) Documents cités:
- EP-A- 0 239 969
- EP-A- 0 421 406
- US-A- 4 803 049
- US-A- 5 308 754
- DATABASE WPI Section Ch, Week 8909 Derwent Publications Ltd., London, GB; Class A85, AN 89-063369 XP002055319 & JP 01 013183 A (CANON KK) , 18 janvier 1989

## Description

### Domaine technique

La présente invention concerne un support d'électrodes comportant au moins une électrode recouverte par un dépôt ainsi qu'un système de lecture de ce support.

L'invention s'applique notamment à la fabrication de capteurs ou d'autres éléments sensibles miniaturisés dans lesquels des puces munies d'un grand nombre d'électrodes doivent être réalisées.

Ces électrodes, pour être adaptées à leurs fonctions spécifiques dans le capteur ou l'élément sensible, doivent être recouvertes de matériaux appropriés.

A titre d'exemple, l'invention s'applique à la réalisation d'éléments miniaturisés tels que des « biochips » qui sont des puces comportant une partie de circuit électrique réalisée sur un substrat, tel qu'un champ d'électrodes, et une partie biologique réalisée à la surface de la puce.

Dans cet exemple, il convient de déposer sélectivement sur les électrodes des composés chimiques compatibles avec les produits biologiques.

### Etat de la technique antérieure

On connaît des supports d'électrodes qui sont réalisés en silicium et comportent des éléments actifs permettant, par un adressage approprié, un dépôt électrochimique sur ces électrodes.

De plus, les systèmes associés, qui permettent de lire ce qui est déposé sur les électrodes, utilisent une source lumineuse, qui est apte à éclairer la face supérieure des électrodes, et des moyens de détection de lumière qui sont placés en regard des électrodes et associés à des moyens optiques.

Le problème essentiel posé par de telles structures est celui de leur coût.

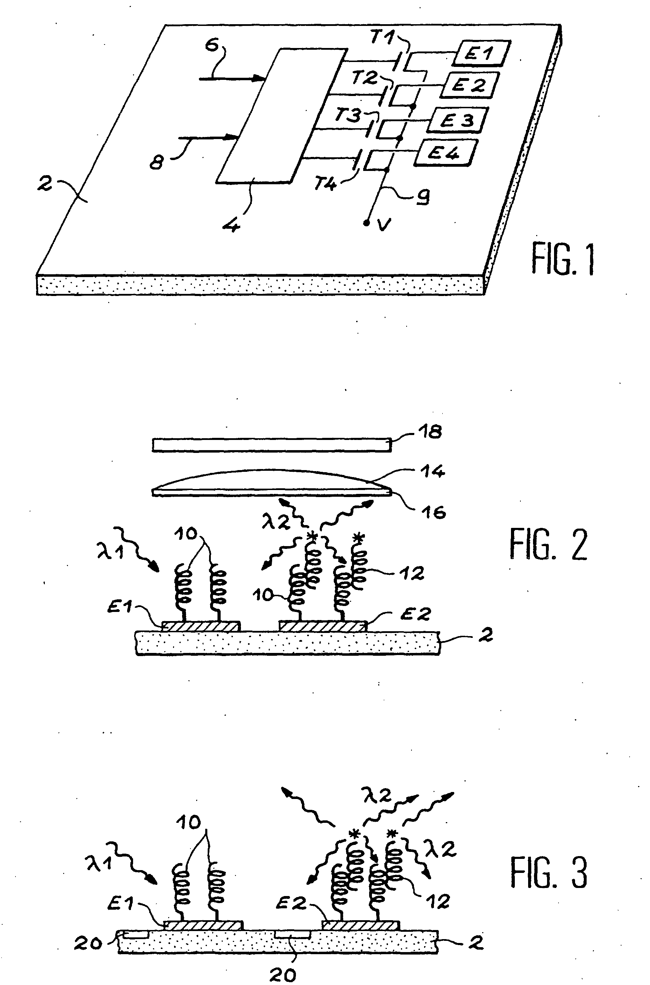

Tout ceci est schématiquement illustré par les figures 1 à 3 des dessins annexés.

On voit sur la figure 1 un substrat 2 par exemple en silicium.

La surface de ce substrat 2 est rendue fonctionnelle par greffage de molécules-sondes.

De plus, cette surface est rendue sélectivement fonctionnelle par adressage sélectif d'une électrode choisie parmi une pluralité d'électrodes indépendantes E1, E2, E3 et E4, par exemple l'électrode E2, les autres électrodes n'étant pas sélectionnées.

L'adressage d'une électrode correspond à l'application à celle-ci d'une tension différente de la tension de référence d'un bain non représenté, dans lequel les molécules-sondes sont diluées.

Cette tension différente de la tension de référence doit être suffisamment élevée pour provoquer soit un phénomène d'électrodéposition (dans le cas d'une utilisation de polymères conducteurs du type polypyrrol ou polyaniline qui sont porteurs des molécules-sondes), soit un transfert de charges irréversible (dans le cas d'une réaction de greffage covalent).

Cet adressage est obtenu en réalisant un circuit de commande 4 sur la surface du substrat 2 où sont les électrodes.

Dans l'exemple de la figure 1, ce circuit de commande 4 est un démultiplexeur qui reçoit des données de poids fort et des données de poids faible par l'intermédiaire de lignes électriques 6 et 8 et qui commande les quatre électrodes indépendantes E1, E2, E3 et E4, respectivement par l'intermédiaire de quatre transistors T1, T2, T3 et T4.

On voit également sur la figure 1 une ligne 9 qui permet d'appliquer une tension V aux sources des transistors de sortie du démultiplexeur 4.

Cette tension V sert à polariser les électrodes sélectionnées grâce à ce démultiplexeur 4.

La lecture des électrodes ou plus exactement de ce qui est déposé sur celles-ci s'effectue par voie optique
- soit en utilisant un système optique comme l'illustre schématiquement la figure 2,
- soit en intégrant des photodétecteurs sur le substrat 2 comme l'illustre schématiquement la figure 3.

Sur la figure 2, on voit le substrat 2 en coupe transversale schématique et partielle ainsi que seulement deux des électrodes E1 à E4, par exemple l'électrode E1 et l'électrode E2.

Ces électrodes E1 à E4 portent toutes des molécules-sondes 10.

Après l'étape de réalisation des dépôts électrochimiques, le support avec les électrodes est recouvert par un analyte contenant des molécules-cibles aptes à être hybridées sur certaines des molécules-sondes.

La lecture de l'hybridation est alors réalisée soit directement en présence de l'analyte soit après élimination de ce dernier comme dans les exemples représentés sur les figures 2 et 3.

Ainsi certaines de ces molécules-sondes, par exemple celles de l'électrode E2, sont hybridées à des branches d'ADN 12 tandis que les autres ne le sont pas.

Ces branches d'ADN 12 sont marquées par un produit de marquage fluorescent de telle manière que, lorsque les branches d'ADN ainsi marquées sont éclairées au moyen d'une lumière de longueur d'onde λ1 provenant d'une source lumineuse non représentée, ces branches marquées émettent une lumière ayant une autre longueur d'onde λ2.

Une lentille 14, qui est munie d'un filtre optique 16 capable d'arrêter la lumière de longueur d'onde λ1 et de laisser passer la lumière de longueur d'onde λ2 est disposée au-dessus de la surface du substrat 2, en regard des électrodes.

Des moyens de détection 18, qui comprennent par exemple des dispositifs à couplage de charges, sont disposés au-dessus de la lentille 14, cette lentille 14 étant ainsi comprise entre les moyens de détection 18 et la surface du substrat 2.

Ces moyens de détection 18 permettent de détecter la lumière de longueur d'onde λ2 qui est focalisée sur les dispositifs à couplage de charges.

Dans la variante schématiquement et partiellement illustrée par la figure 3, on utilise des photodétecteurs 20 centrés sur la longueur d'onde λ2, intégrés au substrat 2 et respectivement placés près des électrodes.

Comme on l'a vu plus haut, le principal problème des systèmes schématiquement illustrés par les figures 1 à 3 est un problème de coût, à savoir :
- le coût du « biochip » réalisé sur du silicium,
- le coût de la détection soit par un système optique (figure 2), soit par des détecteurs intégrés (figure 3).

Par ailleurs, dans le cas d'une lecture en présence de l'analyte, la lecture est perturbée par la présence de l'analyte.

### Exposé de l'invention

La présente invention a pour but de remédier aux inconvénients précédents en proposant d'utiliser un substrat et des électrodes qui sont transparents à la lumière destinée à l'excitation des branches d'ADN marquées et/ou à la lumière émise par ces dernières.

Par exemple, la source lumineuse qui émet cette lumière peut être placée sous le substrat de sorte que les moyens de détection peuvent être disposés suffisamment près des électrodes pour ne plus avoir besoin du système optique (lentille plus filtre) ou inversement.

De façon précise, la présente invention a pour objet un support d'électrodes, ce support comprenant un substrat et des électrodes qui sont formées sur ce substrat, au moins l'une de ces électrodes étant destinée à être recouverte par un dépôt apte à reconnaître et à capturer des molécules-cibles qui sont sensibles à une première lumière et qui sont capables d'émettre une deuxième lumière distincte de la première lumière lorsque ces molécules-cibles sont excitées par cette première lumière, ce support étant caractérisé en ce que le substrat et les électrodes sont transparents à au moins l'une des première et deuxième lumières.

Le substrat et les électrodes sont transparents à la première lumière lorsque la source de première lumière est placée sous le support et ils doivent être transparents à la deuxième lumière lorsque les moyens de détection de celle-ci sont placés sous le support.

Pour simplifier les éléments optiques nécessaires à l'éclairage des molécules-cibles et à la détection de la lumière émise par celles-ci, on a intérêt à disposer la source et les moyens de détection de part et d'autre du support.

Le choix des différentes dispositions possibles dépend des applications du support.

Dans le cas particulier où la lecture se fait en présence de l'analyte contenant les molécules-cibles pour éviter une détérioration du signal émis par les molécules-cibles due à la présence d'analyte (par exemple absorption/défocalisation), les moyens de détection sont avantageusement placées du côté de la face-arriéré du support.

Pour maintenir l'analyte dans le support, celui-ci forme par exemple une cuvette qui peut être éventuellement fermée par une lame qui doit être transparente à la lumière de la source lorsque celle-ci est placée du côté de la face-avant du support.

Grâce à l'invention on réduit ainsi le coût du support et l'on simplifie le système de lecture de ce support par rapport à l'art antérieur.

De préférence, pour réduire encore plus le coût du support, le substrat est en verre.

Des moyens électroniques peuvent être intégrés au substrat.

Selon un mode de réalisation particulier du support objet de l'invention, ces moyens électroniques comprennent un circuit d'adressage qui est intégré au substrat et destiné à commander les électrodes soit pour effectuer un dépôt électrochimique sélectif soit pour contrôler le phénomène d'hybridation.

Ces moyens électroniques peuvent comporter également des moyens de chauffage.

Au lieu de ce circuit d'adressage ou en plus de celui-ci, le support objet de l'invention peut aussi comprendre un circuit de multiplexage qui est intégré au substrat et destiné au multiplexage de signaux adressés aux électrodes.

De préférence, le circuit d'adressage et/ou le circuit de multiplexage sont en silicium amorphe.

Ceci contribue aussi à réduire le coût du support.

Selon un premier mode de réalisation particulier de l'invention, les zones comprises entre les électrodes comprennent des moyens supplémentaires d'arrêt de la première lumière.

Selon un deuxième mode de réalisation particulier, les zones comprises entre les électrodes comprennent des moyens supplémentaires de contrôle du potentiel électrique au voisinage des électrodes.

Les moyens supplémentaires comprennent de préférence des éléments électriquement conducteurs, opaques à la première lumière, disposés dans les zones comprises entre les électrodes et électriquement isolés de ces électrodes.

En variante, les moyens supplémentaires peuvent comprendre des éléments électriquement isolants, opaques à la première lumière, disposés dans les zones comprises entre les électrodes.

Selon un mode de réalisation particulier de l'invention, le support comporte une cuvette apte à contenir un analyte comprenant des molécules-cibles.

La présente invention concerne aussi un système de lecture du support d'électrodes objet de l'invention, ce système de lecture comprenant :
- une source lumineuse apte à émettre la première lumière pour exciter les molécules-cibles, et
- des moyens de détection de la deuxième lumière.

Selon un mode de réalisation particulier du système objet de l'invention, les électrodes forment une matrice et les moyens de détection comprennent une matrice de détecteurs de la deuxième lumière.

Ces détecteurs peuvent être avantageusement munis d'un filtre apte à absorber ou à réfléchir la première lumière et à laisser passer la deuxième lumière.

Le pas de la matrice de détecteurs peut être égal au pas de la matrice des électrodes ou inférieur à ce pas.

Les moyens de détection peuvent être disposés directement en regard des électrodes et de façon suffisamment proche de celles-ci pour éviter l'utilisation de moyens optiques de focalisation entre les électrodes et les moyens de détection.

En variante, les moyens de détection sont intégrés au substrat ou sont placés sous le substrat.

La source lumineuse apte à émettre la première lumière peut être placée sous le substrat,

Les moyens de détection peuvent comprendre des détecteurs de la deuxième lumière et des moyens de focalisation de celle-ci sur ces détecteurs, ces moyens de focalisation étant disposés entre ces détecteurs et les électrodes.

A titre d'exemple ces moyens de focalisation peuvent comporter un réseau de microlentilles et/ou un réseau de microdiaphragmes qui sont avantageusement disposés sur le support.

### Brève description des dessins

La présente invention sera mieux comprise à la lecture de la description d'exemples de réalisation donnés ci-après, à titre purement indicatif et nullement limitatif, en faisant référence aux dessins annexés sur lesquels :
- la figure 1, déjà décrite, est une vue en perspective schématique d'un support d'électrodes connu,
- la figure 2, déjà décrite, est une vue en coupe schématique d'un support d'électrodes connu, associé à un système connu de lecture des électrodes,
- la figure 3, déjà décrite, est une vue en coupe schématique d'une variante connue du système de lecture de la figure 2,
- la figure 4 est une vue en perspective schématique d'un mode de réalisation particulier du support d'électrodes objet de l'invention,
- la figure 5 est une vue en perspective schématique d'un support d'électrodes conforme à l'invention,
- la figure 6 est une vue en coupe transversale schématique du support de la figure 5,
- la figure 7 illustre schématiquement un mode d'adressage des électrodes d'un support conforme à l'invention,
- la figure 8 illustre schématiquement un dépôt électrochimique sur des électrodes d'un support conforme à l'invention,
- la figure 9 illustre schématiquement un dépôt électrochimique sur des électrodes d'un autre support conforme à l'invention,
- la figure 10 illustre schématiquement un dépôt électrochimique sur des électrodes d'un autre support conforme à l'invention,
- la figure 11 est une vue en coupe schématique d'un système de lecture conforme à l'invention,
- les figures 12 et 13 sont des vues en coupe schématiques d'autres systèmes de lecture conformes à l'invention, et
- la figure 14 est une vue en coupe transversale schématique d'un autre support conforme à l'invention.

### Exposé détaillé de modes de réalisation particuliers

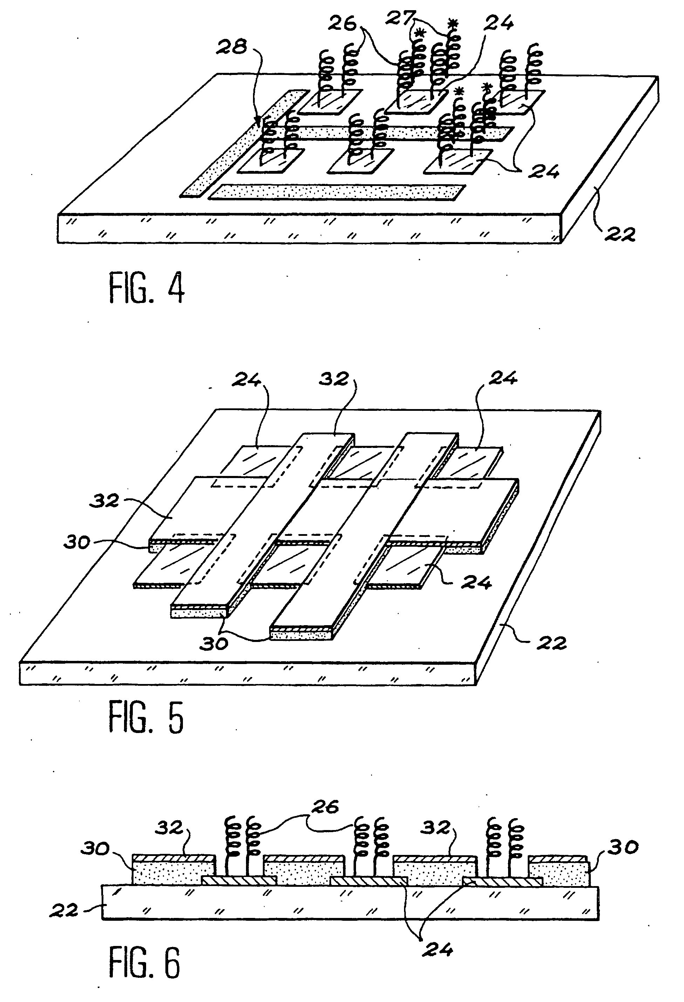

Le support d'électrodes conforme à l'invention, qui est schématiquement représenté en perspective sur la figure 4, comprend un substrat de verre 22 et une matrice d'électrodes 24 qui sont formées à la surface de ce substrat 22.

Chacune des électrodes 24 est recouverte par un dépôt électrochimique constitué de molécules-sondes plus simplement appelées « sondes ».

Ces sondes sont aptes à reconnaître et à capturer des molécules-cibles qui sont sensibles à une lumière de longueur d'onde λ1.

(On entend par « lumière de longueur d'onde λ » une fenêtre spectrale incluant λ).

Toutes les électrodes 24 portent à leur surface des sondes 26 et certaines de ces sondes ont capturé des molécules-cibles 27 tandis que les autres n'en ont pas capturé.

Lorsque les sondes reçoivent la lumière de longueur d'onde λ1, celles qui ont capturé des molécules-cibles et seulement celles-là émettent par l'intermédiaire des molécules-cibles une lumière de longueur d'onde λ2 qui est différente de λ1 et que l'on peut détecter comme on le verra par la suite

. Conformément à la présente invention, le substrat 22 est en verre de manière à être transparent à la lumière de longueur d'onde λ1 et toutes les électrodes 24 sont faites d'un matériau électriquement conducteur et transparent à cette lumière de longueur d'onde λ1.

Dans l'exemple de la figure 4, le support . d'électrodes conforme à l'invention comprend aussi des moyens électroniques 28 qui sont intégrés au substrat de verre 22 du côté de la surface de ce substrat qui porte les électrodes 24.

Ces moyens électroniques sont par exemple en silicium amorphe.

De plus, ces moyens électroniques consistent en un circuit d'adressage des électrodes destiné à permettre l'étape de fonctionnalisation des électrodes c'est-à-dire le greffage des sondes.

Ce circuit d'adressage peut comporter un circuit de multiplexage suivant la complexité de la matrice d'électrodes.

Le support d'électrodes conforme à l'invention, qui est schématiquement représenté en perspective sur la figure 5 diffère de celui de la figure 4 par le fait qu'il comporte en plus des éléments électriquement isolants 30 qui sont formés sur les zones de la surface du substrat 22, zones qui sont comprises entre les électrodes 24.

Sur ces éléments isolants 30 sont respectivement formées des couches métalliques 32 qui sont opaques à la lumière de la longueur λ1 de manière à arrêter celle-ci.

Les couches métalliques 32 peuvent permettent de contrôler le potentiel électrique au voisinage des électrodes 24.

Elles peuvent former une ou plusieurs contre-électrodes.

Elles forment également une obturation optique de parties non sensibles du support d'électrodes.

Une telle obturation optique permet avantageusement d'améliorer le rapport signal/bruit lors de la lecture optique de la matrice.

La figure 6 est une vue en coupe transversale schématique du support d'électrode de la figure 5.

Dans une variante de réalisation, les éléments isolants 30 sont formés sur le substrat 22.

Ces éléments sont alors avantageusement réalisés dans un matériau opaque à la lumière de longueur λ1 pour réaliser également une obturation optique.

On explique ci-après une méthode d'adressage lors du dépôt électrochimique sur des électrodes du genre de celles qui sont représentées sur les figures 4 et 5.

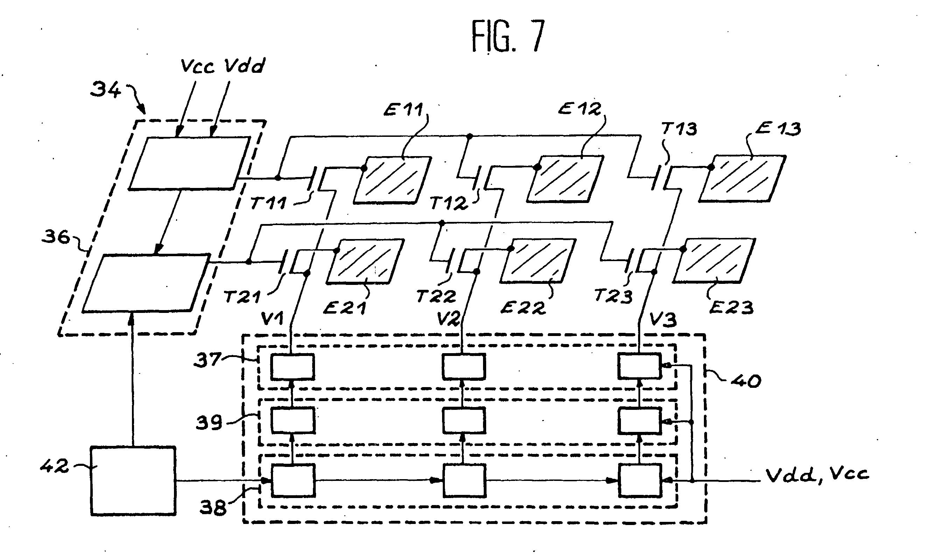

On voit sur la figure 7 une matrice de six électrodes.

Ces électrodes sont référencées E11, E12, E13 pour la première ligne de la matrice et E21, E22 et E23 pour la deuxième ligne de la matrice.

On voit également sur la figure 7 un circuit électronique 34 de commande de ces électrodes.

Ce circuit électronique comprend un registre à décalage 36 ainsi qu'un autre registre à décalage 38.

Ce registre 38 est associé à un registre de transfert parallèle de type « *latch* » 39 combiné à un étage d'amplification à gain 1 de type « *buffer* » 37, l'ensemble constitué par l'étage 37, le registre 38 et le registre 39 ayant la référence 40.

Chaque électrode Eij est associée à un transistor Tij, l'indice i prenant l'une quelconque des valeurs 1 et 2 et l'indice j prenant l'une quelconque des valeurs 1, 2 et 3.

Les deux registres 36 et 38 sont commandés par une horloge 42.

Le registre 36 comporte deux sorties aptes à fournir l'une un signal S1 et l'autre un signal S2 respectivement aux deux lignes de la matrice, par l'intermédiaire des transistors qui lui sont associés.

Plus précisément, les transistors Tij sont des transistors à effet de champ.

Le drain de chaque transistor est relié à l'électrode correspondante.

Les grilles des transistors associés à la première ligne de la matrice sont toutes reliées à la première sortie du registre 36 pour recevoir le signal S1.

Les grilles des transistors associés à la deuxième ligne de la matrice sont toutes reliées à la deuxième sortie du registre 36 pour recevoir le signal S2.

Les sources des deux transistors associés à la première colonne de la matrice d'électrodes sont toutes deux reliées à une première sortie de l'étage d'amplification 37 pour qu'on puisse leur appliquer une tension contrôlée référencée V1.

Les sources des deux transistors associés à la deuxième colonne de la matrice d'électrodes sont toutes deux reliées à une deuxième sortie de l'étage d'amplification 37 pour qu'on puisse leur appliquer une autre tension contrôlée référencée V2, éventuellement distincte de V1.

Les sources des deux transistors associés à la troisième colonne de la matrice d'électrodes sont toutes deux reliées à une troisième sortie de l'étage d'amplification 37 pour qu'on puisse leur appliquer une autre tension contrôlée référencée V3, éventuellement distincte de V1 et de V2.

Sur la figure 7, Vcc et Vdd représentent les tensions d'alimentation des registres 36 et 38.

On peut ainsi faire l'adressage des électrodes de façon séquentielle, ligne par ligne, en utilisant la structure matricielle de ces électrodes comme cela se fait dans les matrices de TFT, qui sont destinées à l'affichage.

Chacune des tensions V1, V2 et V3 est susceptible de prendre des valeurs qui sont soit inférieures au seuil de tension caractéristique Vs de la fonctionnalisation sélective (électrode non sélectionnée) soit supérieures à ce seuil (électrode sélectionnée).

Le transfert de charge n'intervient que lorsque les signaux de sélection S1, S2 sont supérieurs au seuil des transistors, ce qui est une condition pour que les transistors soient passants (état « on »).

Par exemple, le transistor T11 est non sélectionné lorsque V1 est inférieure à la tension de seuil Vs bien que le signal S1 soit « on » et il n'y a alors pas de dépôt sur l'électrode correspondante.

Le transistor T11 est également non sélectionné lorsque V1 est supérieure à la tension Vs mais S1 est « off » c'est-à-dire inférieure au seuil du transistor.

Dans le cas où simultanément S1 est « on » et V1 est supérieure à Vs, le dépôt se produit sur l'électrode E11 associé au transistor T11.

Ce mode de sélection, bien connu dans le domaine de l'affichage, s'applique directement ici pour rendre sélectivement fonctionnelles les électrodes formées à la surface d'un substrat tel que le substrat 22 qui, dans les exemples représentés sur les figures 4 à 6, constitue un « biochip ».

On explique ci-après la réalisation des signaux S1, S2, V1, V2 et V3.

Ces signaux sont obtenus de la façon expliquée ci-après, qui est connue pour les afficheurs.

Les signaux S1 et S2 résultent du registre à décalage 36 de la figure 4.

Les signaux de tension V1, V2 et V3 sont issus des tensions Vcc et Vdd par l'intermédiaire de l'ensemble 40, un transfert d'informations ayant lieu simultanément lors de la commutation du registre à décalage 36 et du registre de transfert parallèle 39.

Ces fonctions sont réalisables :
- soit par une électronique extérieure (connexions amovibles sur les électrodes)
- soit en réalisant les circuits électroniques en silicium amorphe à la surface du substrat de verre 22 qui porte les électrodes (le procédé de fabrication étant alors identique à celui des transistors associés à la matrice d'électrodes).

Il est à noter que la fréquence de commutation des circuits électroniques peut être faible (environ quelques kHz) et donc compatible avec la mobilité électronique dans le silicium amorphe.

Le procédé de fabrication mentionné ci-dessus permet de traiter de façon industrielle des plaques de verre d'environ 500 µm d'épaisseur pour 500 mm de côté, le coût de fabrication étant ainsi inférieur au coût de fabrication des supports d'électrodes de l'art antérieur.

On explique ci-après les modes de dépôt.

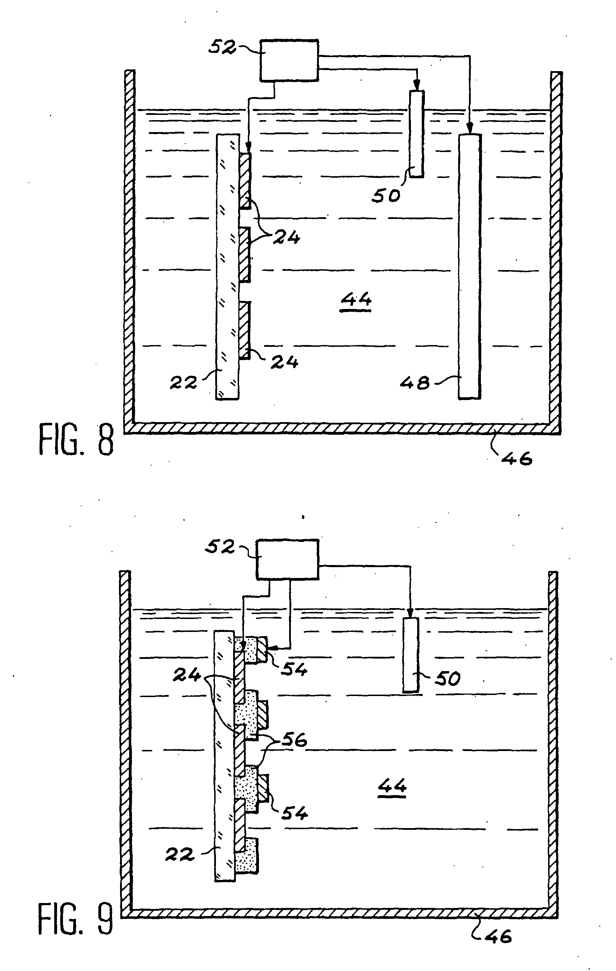

On voit sur la figure 8 le support d'électrode comprenant le substrat de verre 22 et les électrodes 24.

Ce support d'électrodes est plongé dans un bain 44, par exemple un bain de polypyrrole, qui est contenu dans un récipient 46.

En regard des électrodes 24, se trouve une contre-électrode 48 également plongée dans ce bain 44.

On peut utiliser également une électrode de référence 50 que l'on place également dans le bain.

Les électrodes 24 ainsi que l'électrode 48 et l'électrode 50 sont reliées à un potentiostat 52 pour réaliser les dépôts sélectifs sur les électrodes 24.

La figure 9 illustre schématiquement la possibilité de réaliser la contre-électrode sur le substrat 22 par exemple sous la forme d'une pluralité de petites électrodes 54 respectivement associées aux électrodes 24.

Dans ce cas, les électrodes 54 sont des couches métalliques respectivement formées sur des couches isolantes 56 elles-mêmes respectivement formées au voisinage des électrodes 24 comme on le voit sur la figure 9.

Lors du dépôt il se produit un transfert de charges de chaque électrode 24 à l'électrode associée 54.

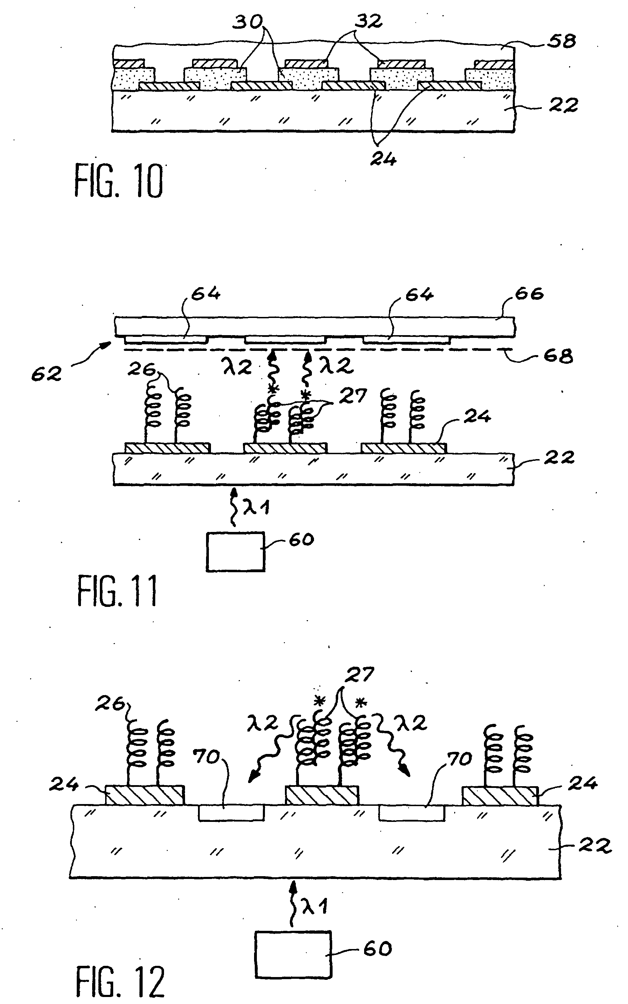

La figure 10 illustre schématiquement la possibilité d'utiliser, en tant que contre-électrode, l'ensemble des couches métalliques dont il a été question dans la description des figures 5 et 6.

Il s'agit alors d'un mode de dépôt simplifié permettant de limiter le volume du bain.

On voit sur cette figure 10 les électrodes 24 formées sur le substrat 22 ainsi que les couches métalliques 32 respectivement formées sur les couches isolantes 30.

Dans l'exemple de la figure 10, il n'y a pas d'électrode de référence.

Il est à noter que l'utilisation d'une électrode de référence est d'autant plus justifiée que le volume du bain est important.

Sur la figure 10, la référence 58 représente le bain.

On explique ci-après la lecture du support d'électrodes de la figure 4.

La lecture de celui-ci est expliquée en faisant référence à la figure 11 où l'on voit en coupe transversale le substrat de verre 22 portant les électrodes 24 qui sont respectivement associées à des molécules-sondes 26 dont certaines portent une molécule-cible marquée 27 tandis que les autres n'en portent pas.

Le système de lecture permettant la lecture du support des électrodes 24 comprend une source lumineuse 60 destinée à émettre la lumière de longueur d'onde λ1.

Cette source 60 est placée sous le substrat de verre 22 comme on le voit sur la figure 11.

Les molécules-sondes qui sont marquées ou, plus exactement, qui portent une molécule-cible marquée et qui reçoivent cette lumière de longueur d'onde λ1 émettent à leur tour une lumière de longueur d'onde λ2.

Le système de lecture comprend également des moyens 62 de détection de la lumière de longueur d'onde λ2.

Ces moyens de détection 62 comprennent une matrice 64 de photodétecteurs appropriés par exemple des détecteurs à couplage de charges (CCD).

Cette matrice 64 de photodétecteurs est formée sur un substrat approprié 66.

Les moyens de détection 62 comprennent également un filtre sélectif 68 placé en regard des détecteurs à couplage de charges et donc interposé entre ces détecteurs et les électrodes 24 comme on le voit sur la figure 11.

Compte tenu de l'éclairage par la face-arrière du substrat 22, on est en mesure de placer les moyens de détection 62 de façon suffisamment proche des molécules-sondes pour ne plus avoir besoin de moyens optiques de focalisation entre les électrodes et la matrice de photodétecteurs.

Au lieu d'une matrice de détecteurs à couplage de charges (CCD) on pourrait utiliser une matrice de détection de photons de type CMOS.

Le pas de la matrice de photodétecteurs 64 peut être égal au pas de la matrice des électrodes 24.

On réalise par exemple le filtre sélectif 68 au moyen de couches minces (par exemple au moyen de multicouches d'oxyde de silicium, titane dont les indices de réfraction sont différents les uns des autres).

Le filtre sélectif 68 laisse passer la lumière de longueur d'onde λ2 et absorbe ou réfléchit la lumière de longueur d'onde λ1.

L'utilisation des couches 30 ou 32 opaques à λ1 est un mode de réalisation avantageux de la présente invention car, grâce à ces couches opaques, il y a moins de lumière parasite que dans l'art antérieur.

En variante, le pas des photodétecteurs peut être inférieur au pas des électrodes (et donc au pas des groupes de molécules-sondes), ce qui permet d'éviter un positionnement précis de ces photodétecteurs par rapport aux molécules-sondes.

La figure 12 illustre schématiquement la possibilité de réaliser la matrice de photodétecteurs dans le substrat de verre, au niveau des électrodes.

On voit sur la figure 12, ce substrat de verre 22 muni de la matrice d'électrodes 24.

Les photodétecteurs alors référencés 70 sont intégrés au substrat de verre 22.

On voit encore les molécules-sondes 26 qui sont greffées sur les électrodes 24 et dont certaines portent une molécule-cible marquée.

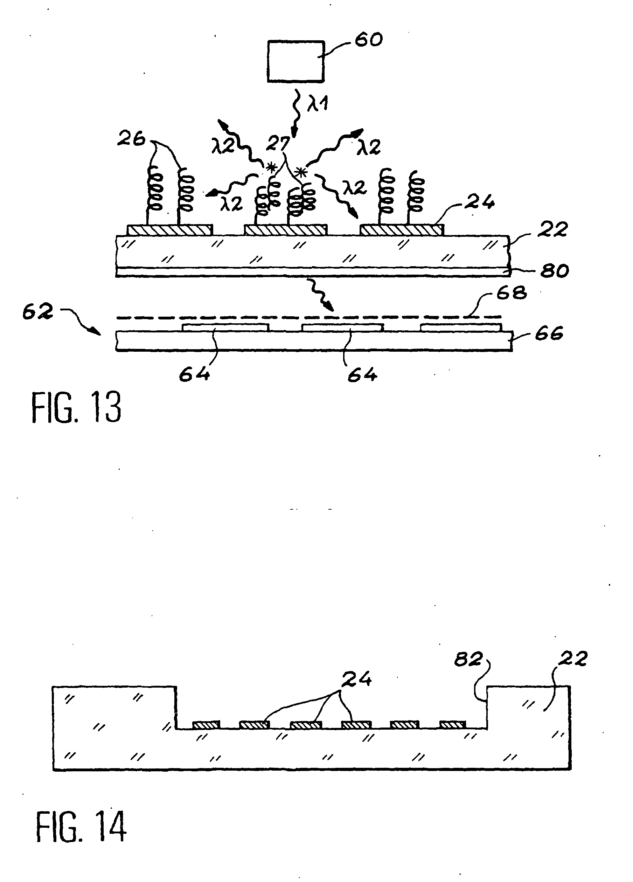

La figure 13 illustre schématiquement une variante de l'invention.

Dans le cas de la figure 13, le substrat 22 est transparent à la lumière de longueur d'onde λ2.

La source 60 est placée au-dessus du substrat 22, en regard des électrodes 24.

Les moyens de détection 62 de la figure 11 sont également utilisés dans le cas de la figure 13 mais sont placés en dessous du substrat 22 de telle manière que la matrice de photodétecteurs 64 reçoive la lumière de longueur d'onde λ2.

Dans le cas de la figure 13, ces moyens de détection 62 comprennent, en outre, des moyens 80 de focalisation qui sont disposés entre les photodétecteurs et les électrodes 24 et prévus pour focaliser la lumière de longueur d'onde λ2 sur les photodétecteurs.

Ces moyens de focalisation peuvent comprendre un réseau de microlentilles ou un réseau de microdiaphragmes ou les deux, ces microlentilles et/ou ces microdiaphragmes étant formés sur la face-arrière du substrat 22.

Dans les exemples de l'invention schématiquement illustrés par les figures 4 à 13, on a supposé que la lecture de l'hybridation des molécules-cibles sur les molécules-sondes avait lieu après élimination de l'analyte contenant les molécules-cibles.

La figure 14 illustre schématiquement la possibilité de réaliser cette lecture en présence de l'analyte.

On voit que le substrat 22 comprend alors une cuvette 82 qui est apte à contenir l'analyte contenant les molécules-cibles et au fond de laquelle se trouvent les électrodes 24.

Le substrat peut être formé d'un seul élément (cas de la figure 14) ou de plusieurs éléments dont l'assemblage permet de constituer la cuvette 82.

## Revendications

1. Support d'électrodes, ce support comprenant un substrat (22) et des électrodes (24) qui sont formées sur ce substrat, au moins l'une de ces électrodes étant destinée à être recouverte par un dépôt apte à reconnaître et à capturer des molécules-cibles qui sont sensibles à une première lumière et qui sont capables d'émettre une deuxième lumière distincte de la première lumière lorsque ces molécules-cibles sont excitées par cette première lumière, ce support étant **caractérisé en ce que** le substrat et les électrodes sont transparents à au moins l'une des première et deuxième lumières.

2. Support selon la revendication 1, dans lequel le substrat est en verre.

3. Support selon l'une quelconque des revendications 1 et 2, dans lequel des moyens électroniques (28) sont intégrés au substrat.

4. Support selon la revendication 3, dans lequel ces moyens électroniques (28) comprennent un circuit d'adressage qui est intégré au substrat et destiné à commander les électrodes.

5. Support selon l'une quelconque des revendications 1 à 4, comprenant en outre un circuit de multiplexage qui est intégré au substrat (22) et destiné au multiplexage de signaux adressés aux électrodes (24).

6. Support selon l'une quelconque des revendications 4 et 5, dans lequel le circuit est en silicium amorphe.

7. Support selon l'une quelconque des revendications 1 à 6, dans lequel les zones comprises entre les électrodes (24) comprennent des moyens supplémentaires (32) d'arrêt de la première lumière.

8. Support selon l'une quelconque des revendications 1 à 7, dans lequel les zones comprises entre les électrodes (24) comprennent des moyens supplémentaires (32) de contrôle du potentiel électrique au voisinage des électrodes.

9. Support selon l'une quelconque des revendications 7 et 8, dans lequel les moyens supplémentaires comprennent des éléments électriquement conducteurs (32), opaques à la première lumière, disposés dans les zones comprises entre les électrodes et électriquement isolés de ces électrodes.

10. Support selon la revendication 7, dans lequel les moyens supplémentaires comprennent des éléments électriquement isolants, opaques à la première lumière, dsposés dans les zones comprises entre les électrodes.

11. Support selon l'une quelconque des revendications 1 à 10, dans lequel le support comporte une cuvette apte à contenir un analyte comprenant des molécules-cibles.

12. Système de lecture du support selon l'une quelconque des revendications 1 à 11, ce système de lecture comprenant :
- une source lumineuse (60) apte à émettre la première lumière pour exciter les molécules-cibles, et
- des moyens (62) de détection de la deuxième lumière.

13. Système selon la revendication 12, dans lequel les électrodes (24) forment une matrice et les moyens de détection comprennent une matrice de détecteurs (64, 70) de la deuxième lumière.

14. Système selon la revendication 13, dans lequel ces détecteurs sont munis d'un filtre (68) apte à absorber ou à réfléchir la première lumière et à laisser passer la deuxième lumière.

15. Système selon la revendication 13, dans lequel le pas de la matrice de photodétecteurs (64) est égal au pas de la matrice des électrodes (24).

16. Système selon la revendication 13, dans lequel le pas de la matrice de détecteurs est inférieur au pas de la matrice des électrodes.

17. Système selon l'une quelconque des revendications 12 à 16, dans lequel les moyens de détection (62) sont disposés directement en regard des électrodes et de façon suffisamment proche de celles-ci, pour éviter l'utilisation de moyens optiques de focalisation entre les électrodes et les moyens de détection.

18. Système selon l'une quelconque des revendications 12 à 16, dans lequel les moyens de détection sont intégrés au substrat (22).

19. Système selon l'une quelconque des revendications 12 à 16, dans lequel les moyens de détection (62) sont placés sous le substrat.

20. Système selon l'une quelconque des revendications 12 à 19, dans lequel la source lumineuse (60) est placée sous le substrat (22).

21. Système selon la revendication 19, dans lequel les moyens de détection (62) comprennent des détecteurs (64) de la deuxième lumière et des moyens (80) de focalisation de celle-ci sur ces détecteurs, ces moyens de focalisation étant disposés entre ces détecteurs et les électrodes.

## Patentansprüche

1. Elektrodenträger, wobei dieser Träger ein Substrat (22) und auf diesem Substrat ausgebildete Elektroden (24) umfasst und wenigstens eine dieser Elektroden dazu bestimmt ist, mit einem Überzug beschichtet zu werden, der fähig ist, Target-Moleküle zu erkennen und einzufangen, die empfindlich sind für ein erstes Licht und fähig sind, ein sich von dem ersten Licht unterscheidendes zweites Licht zu emittieren, wenn sie, die Target-Moleküle, durch dieses erste Licht angeregt werden,
**dadurch gekennzeichnet,**
**dass** das Substrat und die Elektrode für wenigstens eines der beiden Lichter durchlässig sind.

2. Träger nach Anspruch 1, bei dem das Substrat aus Glas ist.

3. Träger nach einem der Ansprüche 1 und 2, bei dem elektronische Einrichtungen (28) in das Substrat integriert sind.

4. Träger nach Anspruch 3, bei dem diese elektronischen Einrichtungen (28) eine Adressierschaltung umfassen, die in das Substrat integriert ist und dazu dient, die Elektroden zu steuern.

5. Träger nach einem der Ansprüche 1 bis 4, der außerdem eine Multiplexschaltung umfasst, die in das Substrat (22) integriert ist und dazu dient, die an die Elektroden (24) adressierten Signale zu multiplexen.

6. Träger nach einem der Ansprüche 4 und 5, bei dem die Schaltung aus amorphem Silicium ist.

7. Träger nach einem der Ansprüche 1 bis 6, bei dem die zwischen den Elektroden (24) enthaltenen Zonen zusätzliche Sperreinrichtungen (32) für das erste Licht umfassen.

8. Träger nach einem der Ansprüche 1 bis 7, bei dem die zwischen den Elektroden (24) enthaltenen Zonen zusätzliche Sperreinrichtungen (32) zur Steuerung des elektrischen Potentials in der Nähe bzw. Umgebung der Elektroden umfassen.

9. Träger nach einem der Ansprüche 7 und 8, bei dem die zusätzlichen Einrichtungen leitfähige elektrische Elemente (32) umfassen, undurchlässig für das erste Licht und angeordnet in den zwischen den Elektroden enthaltenen Zonen und elektrisch von diesen Elektroden isoliert.

10. Träger nach Anspruch 7, bei dem die zusätzlichen Einrichtungen elektrisch isolierende Elemente umfassen, undurchlässig für das erste Licht und angeordnet in den zwischen den Elektroden enthaltenen Zonen.

11. Träger nach einem der Ansprüche 1 bis 10, bei dem der Träger eine Schale umfasst, die ein Analyt aufnehmen kann, das Target-Moleküle enthält.

12. System zum Lesen des Trägers nach einem der Ansprüche 1 bis 11, wobei dieses Lesesystem umfasst:
- eine Lichtquelle (60), fähig das erste Licht zu emittieren, um die Target-Moleküle anzuregen, und
- Einrichtungen (62) zur Detektion des zweiten Lichts.

13. System nach Anspruch 12, bei dem die Elektroden (24) eine Matrix bilden und die Detektionseinrichtungen eine Matrix von Detektoren (64, 70) des zweiten Lichts umfassen.

14. System nach Anspruch 13, bei dem diese Detektoren mit einem Filter (68) ausgestattet sind, der das erste Licht absorbiert oder reflektiert und das zweite Licht durchlässt.

15. System nach Anspruch 13, bei dem die Teilung der Matrix der Fotodetektoren (64) gleich der Teilung der Matrix der Elektroden (24) ist.

16. System nach Anspruch 13, bei dem die Teilung der Fotodetektorenmatrix kleiner ist als die Teilung der Elektrodenmatrix.

17. System nach einem der Ansprüche 12 bis 16, bei dem die Detektionseinrichtungen (62) den Elektroden direkt und ausreichend nahe gegenüberstehen, um die Verwendung von optischen Fokussiereinrichtungen zwischen den Elektroden und den Detektionseinrichtungen zu vermeiden.

18. System nach einem der Ansprüche 12 bis 16, bei dem die Detektionseinrichtungen in das Substrat (22) integriert sind.

19. System nach einem der Ansprüche 12 bis 16, bei dem die Detektionseinrichtungen (62) sich unter dem Substrat befinden.

20. System nach einem der Ansprüche 12 bis 19, bei dem die Lichtquelle (60) sich unter dem Substrat (22) befindet.

21. System nach Anspruch 19, bei dem die Detektionseinrichtungen (62) Detektoren (64) des zweiten Lichts und Fokussiereinrichtungen (80) dieses Lichts auf diese Detektoren umfassen, wobei diese Fokussiereinrichtungen zwischen diesen Detektoren und den Elektroden angeordnet sind.

## Claims

1. Electrode support, this support comprising a substrate (22) and electrodes (24) that are formed on this substrate, at least one of these electrodes being designed to be covered by a deposit capable of recognizing and capturing molecule-targets that are sensitive to a first light and that are capable of emitting a second light distinct from the first light when these molecule-targets are excited by the first light, this support being **characterized by** the fact that the substrate and electrodes are transparent to at least the first or the second light.

2. Support according to claim 1, wherein the substrate is made of glass.

3. Support according to either of the claims 1 and 2, wherein the electronic means (28) are integrated into the substrate.

4. Support according to claim 3, wherein these electronic means (28) comprise an addressing circuit that is integrated into the substrate and is designed to control the electrodes.

5. Support according to any one of the claims 1 to 4, also comprising a multiplexing circuit that is integrated into the substrate (22) and is designed for multiplexing signals addressed to electrode (24).

6. Support according to either of the claims 4 and 5, wherein the circuit is made of amorphous silicon.

7. Support according to any one of the claims 1 to 6, wherein the areas between the electrodes (24) comprise additional means (32) to stop the first light.

8. Support according to any one of the claims 1 to 7, wherein the areas between the electrodes (24) comprise additional means (32) for controlling the electrical potential in the vicinity of electrodes.

9. Support according to either of the claims 7 and 8, wherein the additional means comprise electrically conducting elements (32) opaque to the first light, placed in the areas between the electrodes and electrically insulated from these electrodes.

10. Support according to claim 7, wherein the additional means comprise electrically insulated elements opaque to the first light, placed in the areas between the electrodes.

11. Support according to any one of the claims 1 to 10, wherein the support comprises a dish suitable for containing an analyte containing molecule-targets.

12. System for reading from the support according to any one of the claims 1 to 11, this reading system comprising:
- a light source (60) capable of emitting first light to excite molecule-targets, and
- means (62) of detecting the second light.

13. System according to claim 12, wherein the electrodes (24) form a matrix and detection means comprise a matrix of detectors (64, 70) of the second light.

14. System according to claim 13, wherein these detectors are fitted with a filter (68) capable of absorbing or reflecting the first light and allowing the second light to pass.

15. System according to claim 13, wherein the pitch of the photodetectors matrix (64) is equal to the pitch of the electrodes matrix (24).

16. System according to claim 13, wherein the pitch of the detectors matrix is less than the pitch of the electrodes matrix.

17. System according to any one of the claims 12 to 16, wherein the detection means (62) are placed directly facing the electrodes, and sufficiently close to them to prevent the use of optical focusing means between the electrodes and detection means.

18. System according to any one of the claims 12 to 16, wherein the detection means are integrated in the substrate (22).

19. System according to any one of the claims 12 to 16, wherein the detection means (62) are placed under the substrate.

20. System according to any one of the claims 12 to 19, wherein the light source (60) is placed under the substrate (22).

21. System according to claim 19, wherein the detection means (62) comprise detectors (64) of the second light and means (80) of focusing this light on these detectors, these focusing means being placed between these detectors and the electrodes.
